# EUROPEAN PATENT SPECIFICATION

(11) **EP 2 196 446 B1**
(45) Date of publication and mention of the grant of the patent: **24.06.2015**
(21) Application number: 09177787.0
(22) Date of filing: 02.12.2009
(51) Int. Cl.: B01J 19/00, C07C 17/02, C07C 19/045

(54) **Reactor system for preventing fouling in the production of ethylene dichloride**
Reaktorsystem zur Verhinderung von Fouling bei der Herstellung von Ethylendichlorid
Système de réacteur pour la prévention de l'encrassement dans la production de dichlorure d'éthylène

(30) Priority: 15.12.2008 TR 200809473
(43) Date of publication of application: 16.06.2010
(73) Proprietor: Petkim Petrokimya Holding Anonim Sirekti, 35800 Izmir (TR)
(72) Inventor: Ezdesir, Ayhan, 35800 Izmir (TR); Yapici, Erol, Ilkadim Samsun (TR)
(74) Representative: Dericioglu Kurt, Ekin

(56) References cited:
- EP-A1- 0 103 819
- WO-A1-2008/118923
- WO-A2-01/34542
- US-A- 3 328 284
- US-A- 5 110 997
- US-A- 5 240 469
- A.G. BORSA ET AL: "Coke and Byproduct Formation during 1,2-Dichloroethane Pyrolysis in a Laboratory Tubular Reactor" IND.ENG.CHEM., vol. 40, no. 11, 19 April 2001 (2001-04-19), pages 2428-2436, XP002571853

## Description

### Field of the invention

The present invention relates to prevention of foulings which occur in chlorine and ethylene feeding ejectors and reactor circulation lines during production of ethylene dichloride.

### Background of the Invention

Vinyl chloride monomer (VCM) is obtained by subjecting high-purity 1,2-dichlor ethane to cracking process at 460 to 480°C. 1,2-dichlor ethane is obtained by methods of direct chlorination reaction of ethylene and chlorine along with catalyst and oxychlorination reaction of oxygen and HCl (hydrochloric acid) beside ethylene in reaction medium.

Undesirable by-products may occur during production of 1,2-dichlor ethane (ethylene dichloride-EDC) by these two methods. These components which occur during direct chlorination and oxychlorination of ethylene are believed to be oligomeric polychlorinated components and oxygenates. These kind of by-products affect the efficiency of vinyl chloride during pyrolysis of 1,2-dichlor ethane and may decrease purity of the vinyl chloride to be obtained. These impurities may affect efficiency of polyvinyl chloride production wherein the vinyl chloride monomer is used as raw material, and product features in a negative way as well.

In the production of 1,2-dichlor ethane, problems occur relating to fouling formation in various regions of the process particularly in EDC distillation trays and in transfer lines. The oligomeric polychlorinated components and oxygenates which are indicated as a reason for fouling formation, cause the production to be interrupted, by affecting the efficiency of the process in a negative way. In order to prevent this, oil soluble methacrylate or acrylate ester compounds comprising various organic sulphonic acids, alkyl arylsulphonate salts and various phenylenediamine groups as antifoulant in EDC production processes are used.

Making the chlorination residues harmless by eliminating or preventing their formation is extremely important because of economic and environmental reasons. Hexachlorobenzene constitutes the greatest component provided within this resultant residue. The fact that it is resistant to bio-accumulation and decomposition in the environment, harmful to human health and cancerogenic (classified by IARC: International Agency for Research on Cancer as Group 2B) indicates how important it is to eliminate it or prevent its formation.

Elimination of the chlorination residue by combusting bears various difficulties. It is required to carry out proper feeding in combustion furnaces, in order to be able to combust the residue in a controlled manner technically. And this can be provided by means of making the residue fluid by melting it at approximately 230°C. In addition to this, the fact that it is corrosive and the control of the corrosive hydrogen chloride that comes out at the end of combustion appears as another problem. Also the residue which is combusted under uncontrolled and inappropriate conditions, leads to formation of undesirable combustion products such as soot, carbon dioxide and carbon monoxide.

Preparing the solution of the residue by dissolving it in a solvent in order to combust the chlorination residue in a controlled manner in combustion furnaces, bears various disadvantages. Limited solubility of the chlorination residue in solvents such as benzene and 1,2-dichloroethane or preparing proper mixtures of additives which are added for enhancing the solubility by adjusting carbon/hydrogen/chlorine ratios and the solubilization process lasts a long time can be given as example.

In ethylene dichloride production line known in the art, there are at least one cracking furnace which conveys the pure EDC to the circulation and the catalyst loading pump (10), and catalyst loading pump EDC line (15). In the production of 1,2-dichlor ethane, feeding to the catalyst loading and circulation pump (10) of the direct chlorination reactor (1) is carried out by means of a line (15) which is installed over EDC feeding line (14) which goes from pure EDC tank (13) to the cracking furnace and the fed EDC mixes to the reactor fluid which is circulated by means of the catalyst loading and circulation pump (10) of the EDC reactor (1) (Figure 1). Existence of this line (15) leads to foulings in the production line. WO01/34542 for example discloses the recycling of EDC to the chlorination reactor after passing through a falling film evaporator. 10

The United States patent document US5324393, an application known in the art, discloses a method which is developed to prevent fouling composed in ethylene dichloride distillation unit. In this method, a chemical application is in question. A special composition is added to the raw material used in feeding, in an amount enough to prevent fouling. The composition comprises (a) 2-15 weight % of an oil soluble polyacrylate ester or methacrylate ester with alcohol radical of the ester group containing from C4-C22 carbon atoms which contains between 0.1-25 mole % of amino alcohol ester groups; (b) 20-40 weight % a phenylene diamine compound and (c) a heavy aromatic solvent.

The European patent document EP0515027, an application known in the art, discloses a method which is developed to prevent fouling composed in ethylene dichloride distillation unit. An antifoulant substance is added to the raw material in an effective amount enough to prevent fouling. This substance consists of (a) an acylated amine and a polyamine (b) magnesium alkyl aryl sulfonate and (c) a mixture of a and b.

The United States patent document US3328283, an application known in the art, discloses use of partial combination of organic sulfonic acid or alkyl aryl sulfonate salts.

The United States patent document US3328284, an application known in the art, discloses use of alkyl arylsulfonate together with oxyalkyl phenolic compound.

The United States patent document US4540837, an application known in the art, discloses elimination of tar-like undesirable by-products which come into existence during raw EDC production occurring at the end of direct and/or oxychlorination process of carbon tetrachloride and/or perchloroethylene or ethylene for example by arising from C1-C3 hydrocarbons, in chlorination processes. It is stated that the composition of the said by-product mixture is especially hexachlorobenzene, hexachlorobutadiene and hexachloroethane and these undesirable residues are made soluble at 100-180°C in hydrocarbon solvent containing chlorine and oxygen described as promoter additives. The chlorination residue which is made soluble can be eliminated later by means of being combusted in combustion furnace without damaging the environment.

The above mentioned patent documents are for minimizing or preventing the fouling problem encountered in the production of EDC (ethylene dichloride), by chemical methods. It is seen in the prior art that no amendment is made in the production lines without the exception of intervention by chemical methods.

### Summary of the Invention

The objective of the present invention is to realize a process which prevents fouling that occurs in chlorine and ethylene feeding ejectors and reactor circulation lines.

Another objective of the present invention is to realize a process which enables to shorten the maintenance times by obviating the foulings that occur in the lines.

A further objective of the present invention is to realize a process which enables to prevent formation of chlorination residues that are harmful in terms of human and environment.

A yet further objective of the present invention is to realize a process which enables continuous circulation of the reaction fluid in the system.

### Detailed Description of the Invention

A reactor system for fouling prevention in the production of ethylene dichloride (100) is illustrated in the accompanying figures, in which:

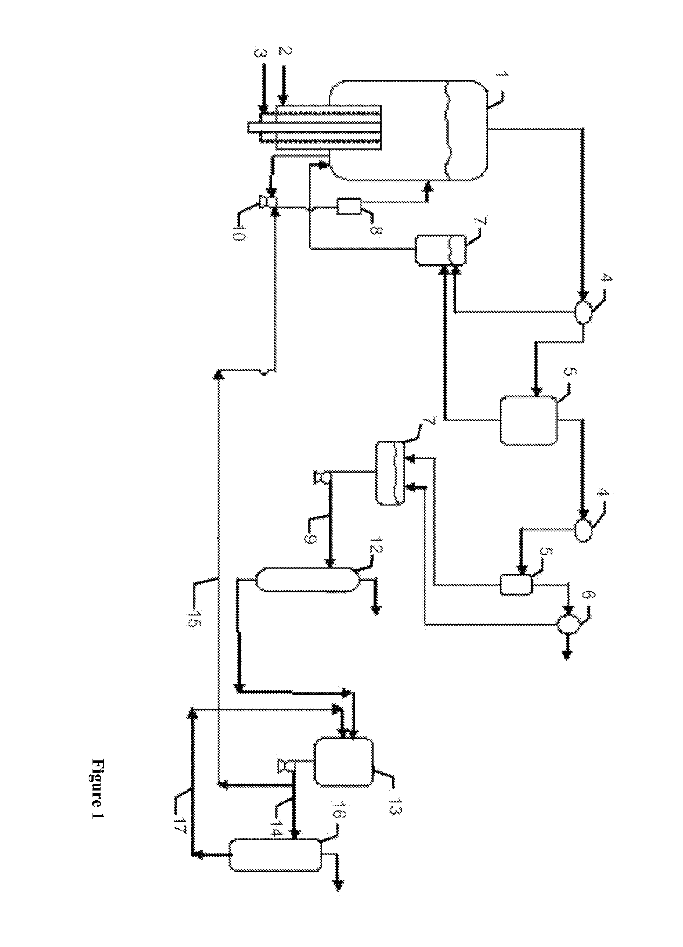

Figure 1 is the schematic view of the 1,2-dichlor ethane production line in prior art.

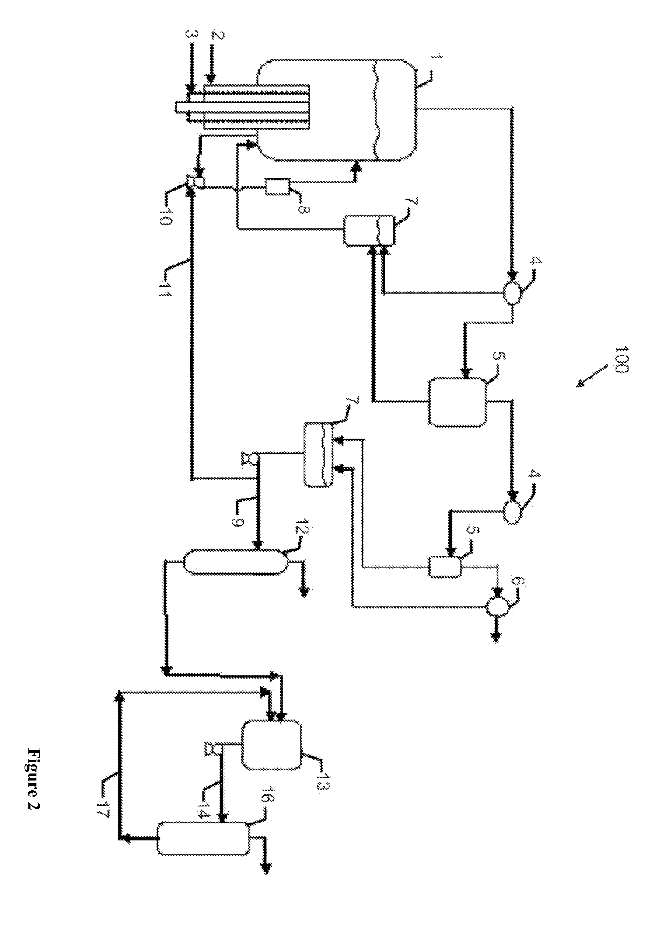

Figure 2 is the schematic view of the 1,2-dichlor ethane production line which prevents fouling.

The parts given in the figures are individually numbered where the numbers refer to the following:
100-A reactor system for fouling prevention in the production of ethylene dichloride
1-Reactor
2-Ethylene
3-Chlorine
4-Condenser
5-EDC separator
6-Vent gas condenser
7-EDC collecting drum
8-Catalyst loading pot
9-EDC line going to the ethylene stripper column
10-Circulation and catalyst loading pump
11-Circulation and catalyst loading pump EDC line
12-Ethylene stripper
13-Pure EDC tank
14- EDC line going to the cracking furnace
15-Circulation and catalyst loading pump of cracking furnace EDC line
16-EDC cracking unit
17-Recycle EDC

A reactor system for fouling prevention in the production of ethylene dichloride (100) essentially comprises
- at least one direct chlorination reactor (1) in the production of 1,2-dichlor ethane,
- at least one ethylene ejector (2) and chlorine ejector (3) which enable raw material input for reaction in the production of 1,2-dichlor ethane,
- at least one condenser (4) which enables to condense the gas outgoing from the reactor (1),
- at least one EDC separator (5) which separates the EDC outgoing from the reactor (1),
- at least one vent gas condenser (6) which enables the gas coming from the EDC separator (5) to condense,
- at least one EDC collecting drum (7) wherein the ethylene dichloride outgoing from the condenser (6) accumulates,
- at least one catalyst loading pot (8) which provides catalyst in order to be used in reaction of the ethylene (2) and the chlorine (3) in the reactor (1),
- at least one EDC line (9) which goes to the ethylene stripper column (12) from the EDC collecting drum (7),
- at least one circulation and catalyst loading pump (10) which pumps catalyst to the catalyst loading pot (8),
- at least one EDC line (11) which conveys the line (9) outgoing from the ethylene stripper column to the circulation and catalyst loading pump (10),
- at least one ethylene stripper (12) which enables the ethylene outgoing from the EDC collecting drum (7) to separate,
- at least one EDC tank (13) wherein the obtained pure ethylene dichloride accumulates,
- at least one EDC line (14) which conveys the pure EDC to the cracking furnace,
- at least one EDC cracking unit (16) which enables the EDC outgoing from the EDC tank (13) to decompose,
- at least one EDC recycle line (17) which sends the EDC outgoing from the EDC cracking unit (16) to the pure EDC tank (13).

In the inventive reactor system for fouling prevention in the production of ethylene dichloride (100), the line (15), which is installed over EDC feeding line (14) from the pure EDC tank (13) to cracking furnace of the direct chlorination reactor catalyst loading and circulation pump (10) of the EDC reactor, is cancelled. Instead of the cancelled line, the feeding of the flow, which comes from the EDC separator (5) and accumulates in the EDC collecting drum (7), is carried out from the EDC line (9) going to the ethylene stripper. Thus for the inventive fouling problem, the circulation and catalyst loading pump EDC line (11) which provides flow to the circulation and catalyst loading pump (10) steps in. The process which is executed over this line obstructs formation of residues elimination of which is difficult, enables to protect human health and environment and prevents foulings which occur in the chlorine ejector (3) and the ethylene feeding ejector (2) and the reactor (1) circulation lines (15), in direct chlorination process.

## Claims

1. A reactor system for fouling prevention in the production of ethylene dichloride (100) **comprising**
- at least one direct chlorination reactor (1) in the production of 1,2-dichlor ethane,
- at least one ethylene ejector (2) and chlorine ejector (3) which enable raw material input for reaction in the production of 1,2-dichlor ethane,
- at least one condenser (4) which enables to condense the gas outgoing from the reactor (1),
- at least one EDC separator (5) which separates the EDC outgoing from the reactor (1),
- at least one vent gas condenser (6) which enables the gas coming from the EDC separator (5) to condense,
- at least one EDC collecting drum (7) wherein the ethylene dichloride outgoing from the condenser (6) accumulates,
- at least one catalyst loading pot (8) which provides catalyst in order to be used in reaction of the ethylene (2) and the chlorine (3) in the reactor (1),
- at least one EDC line (9) which goes to the ethylene stripper column (12) from the EDC collecting drum (7),
- at least one circulation and catalyst loading pump (10) which pumps catalyst to the catalyst loading pot (8),
- at least one ethylene stripper (12) which enables the ethylene outgoing from the EDC collecting drum (7) to separate,
- at least one EDC tank (13) wherein the obtained pure ethylene dichloride accumulates,
- at least one EDC line (14) which conveys the pure EDC to the cracking furnace,
- at least one EDC cracking unit (16) which enables the EDC outgoing from the EDC tank (13) to decompose,
- at least one EDC recycle line (17) which sends the EDC outgoing from the EDC cracking unit (16) to the pure EDC tank (13)
**characterized by**
- at least one circulation and catalyst loading pump EDC line (11) which conveys the line (9) entering the ethylene stripper column to the circulation and catalyst loading pump (10) and enables to prevent fouling in the production of ethylene dichloride.

## Patentansprüche

1. Reaktorsystem zur Verhinderung von Fouling bei der Herstellung von Ethylendichlorid (100) **umfassend**,
- zumindest einen direkten Chlorungs-Reaktor (1) bei der Herstellung von 1,2-dichlorethan,
- zumindest einen Ethylen-Ejektor (2) und Chlor-Ejektor (3), der die Rohstoffeingabe für die Reaktion bei der Herstellung von 1,2-dichlorethan ermöglicht,
- zumindest einen Verdichter (4), der es ermöglicht, die Gasabströmung aus dem Reaktor (1) zu verdichten,
- zumindest einen EDC-Separator (5), der den EDC-Ausgang aus dem Reaktor (1) trennt,
- zumindest einen Entlüftungs-Gasverdichter (6), der es ermöglicht, das aus dem EDC-Separator (5) kommende Gas zu verdichten,
- zumindest einen EDC-Sammelbehälter (7), worin das aus dem Verdichter (6) ausgehende Dichlorethan sich sammelt,
- zumindest einen Katalysatortopf (8), der den Katalysator bereitstellt, der bei der Reaktion von Äthylen (2) und Chlor (3) im Reaktor (1) eingesetzt werden soll,
- zumindest eine EDC-Leitung (9), welche vom EDC-Sammelbehälter (7) zur Abscheider-Kolonne (12) führt,
- zumindest eine Kreislauf-und Katalysatorpumpe (10), welche den Katalysator zum Katalysatortopf (8) pumpt,
- zumindest einen Äthylen-Abscheider (12), der es ermöglicht, dass das aus dem EDC-Sammelbehälter kommende (7) Äthylen getrennt wird,
- zumindest einen EDC-Behälter (13), worin das erhaltene reine Dichlorethan sich anhäuft,
- zumindest eine EDC-Leitung (14), welche das reine EDC zum Spaltofen befördert,
- zumindest eine Cracking-Einheit (16), welche es ermöglicht, das aus dem EDC-Behälter (13) herauskommende EDC zu zersetzen,
- zumindest eine EDC-Rücklaufleitung (17), die das aus der EDC Cracking-Einheit (16) herauskommende EDC zu dem reinen EDC-Behälter (13) überträgt,
**gekennzeichnet durch**
- zumindest eine Kreislauf-und Katalysatorpumpe-EDC-Leitung (11), welche die Leitung (9), die in die Äthylen-Abscheider-Kolonne eingeführt ist, zur Kreislauf-und Katalysatorpumpe (10) befördert und es ermöglicht, das Fouling bei der Herstellung von Dichlorethan zu verhindern.

## Revendications

1. Un système de réacteur pour la prévention de l'encrassement dans la production du dichlorure d'éthylène (100), comportant
- au moins un réacteur de chloration directe (1) dans la production de 1,2-dichlor d'éthane,
- au moins un éjecteur d'éthylène (2) et un éjecteur de chlore (3) permettant à l'entrée de la matière première pour la réaction dans la production de 1,2-dichlor d'éthane,
- au moins un condensateur (4) permettant à la condensation du gaz sortant du réacteur (1),
- au moins un séparateur d'EDC (5) séparant l'EDC sortant du réacteur (1),
- au moins un condensateur des gaz évacués (6) permettant à la condensation du gaz provenant du séparateur d'EDC (5),
- au moins un tambour collecteur d'EDC (7) dans lequel s'accumule du dichlorure d'éthylène provenant du condensateur (6),
- au moins un pot de chargement de catalyseur (8) fournissant le catalyser afin d'être utilisé dans la réaction de l'éthylène (2) et du chlore dans le réacteur (1),
- au moins une ligne d'EDC (9) arrivant à la colonne décapeuse d'éthylène (12) du tambour colleteur d'EDC (7),
- au moins une pompe de circulation et chargement de catalyser (10) pompant du catalyseur au pot de chargement de catalyseur (8),
- au moins un décapeur d'éthylène (12) permettant à la séparation de l'éthylène sortant du tambour collecteur d'EDC (7),
- au moins un réservoir d'EDC (13) dans lequel s'accumule du pur dichlorure d'éthylène obtenu,
- au moins une ligne d'EDC (14) transmettant l'EDC pur au fourneau de craquage,
- au moins une unité de craquage d'EDC (16) permettant à la décomposition de l'EDC sortant du réservoir d'EDC (13),
- au moins une ligne de recyclage d'EDC (17) envoyant l'EDC sortant de l'unité de craquage d'EDC (16) au réservoir d'EDC pur
**caractérisée par**
- au moins une ligne d'EDC de pompe de circulation et chargement de catalyseur (11) transmettant la ligne (9) entrant dans la colonne décapeuse d'éthylène à la pompe de circulation et de chargement de catalyseur (10) et permettant à la prévention de l'encrassement dans la production du dichlorure d'éthylène.
